# EUROPÄISCHE PATENTSCHRIFT

(11) **EP 0 660 735 B1**
(45) Veröffentlichungstag und Bekanntmachung des Hinweises auf die Patenterteilung: **25.02.1998**
(21) Anmeldenummer: 93919018.7
(22) Anmeldetag: 16.09.1993
(51) Int. Cl.: A61N 1/05, A61N 1/39

(54) **IMPLANTIERBARES DEFIBRILLATIONSSYSTEM**
IMPLANTABLE DEFIBRILLATION SYSTEM
SYSTEME DE DEFIBRILLATION IMPLANTABLE

(30) Priorität: 17.09.1992 DE 4231600
(43) Veröffentlichungstag der Anmeldung: 05.07.1995
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-91054 Erlangen (DE)
(74) Vertreter: Christiansen, Henning, Dipl.-Ing.
(86) Internationale Anmeldenummer: DE9300889
(87) Internationale Veröffentlichungsnummer: WO9406507

(56) Entgegenhaltungen:
- EP-A- 0 043 461
- EP-A- 0 064 289
- EP-A- 0 115 778
- EP-A- 0 426 090
- EP-A- 0 453 117
- WO-A-93/02739
- US-A- 4 817 634
- US-A- 4 969 463
- US-A- 5 090 422
- US-A- 5 097 843
- IEEE ENGINEERING IN MEDICINE AND BIOLOGY MAGAZINE, Bd.9, Nr.2, Juni 1990, NEW YORK US Seiten 43 - 49 K.STOKES 'Implantable Pacing Lead Technology'

## Beschreibung

Die Erfindung betrifft ein implantierbares Defibrillationssystem der im Oberbegriff des Anspruchs 1 angegebenen Art.

Das sogenannte Kammerflimmern stellt eine Herzrhythmusstörung dar, welche infolge der ungeordneten Kontraktion einzelner Muskelfasern und der daraus resultierenden unkoordinierten Herzaktivität in kurzer Zeit zum sogenannten plötzlichen Herztod führt, wenn nicht durch geeignete Stimulationsmaßnahmen eine Defibrillation erfolgt. Eine derartige Defibrillation erfolgte zunächst mit entsprechenden, energiereiche Impulse erzeugenden Geräten von extern, wobei zwecks Kardioversion Elektroden am Brustkorb des Patienten angebracht wurden, welche die Impulse in den Thorax einleiteten.

Da derartige Rettungsmaßnahmen mit externen Geräten und durch entsprechend ausgebildete Helfer durchgeführt, für viele Patienten zu spät erfolgten, wurden inzwischen implantierbare Defibrillatoren geschaffen, welche mit Batterien versehen sind und mitsamt der Elektroden in den Körper des Patienten implantiert werden. Wenn zusätzlich Detektionsmittel vorgesehen sind, welche beim Auftreten von Kammerflimmern selbsttätig Defibrillationsimpulse auslösen, so kann der betreffende Patient sich auch außerhalb der Reichweite medizinischer Hilfsmaßnahmen ohne Angst vor einer möglichen Gefährdung durch auftretendes Kammerflimmern bewegen.

Derartige implantierbare Systeme sind in ihrer Effektivität und Verwendungsdauer jedoch noch eingeschränkt durch den hohen Energieverbrauch bei der Defibrillation. Zwar konnte die benötigte Energie bei implantierbaren Geräten gegenüber externen Geraten wesentlich herabgesetzt werden, trotzdem ist die sich ergebende Betriebsdauer insbesondere bei häufig vorkommenden Defibrillationen gering, so daß eine vorzeitige Reimplantation erforderlich ist.

Die Schwierigkeit bei der Wahl geeigneter endothorakischer Elektroden besteht darin, daß die Elektroden möglichst großflächig ausgebildet sein müssen, damit eine Verteilung des Stimulationsstroms auf den gesamten betroffenen Bereich des Herzmuskels erzielt wird. Im Gegensatz zur normalen, schrittmachenden Herzstimulation breiten sich Defibrillationsimpulse nach loklaler Erregung nicht selbsttätig im Herzen aus, sondern es muß dafür gesorgt werden, daß sämtliche zu stimulierenden Bereiche sich im Einwirkungsbereich des Stimulationsstroms befinden.

Aus der DE 26 43 956 C2 ist eine Defibrillationselektrodenanordnung bekannt, mit großflächigen Elektroden bekannt, welche epikardial anzulegen ist und sich an den Umriß des Herzens anformen, wobei eine der Elektroden dem Bereich der Herzspitze und die andere Elektrode dem Bereich des oberen Herzens angeformt ist.

Ungünstig ist bei diesen Elektroden, daß sie immer noch relativ viel Energie benötigen, damit alle erregbaren Zellen des Herzens depolarisiert werden können. Darüber hinweisen die Elektroden noch eine gewisse Eigensteifigkeit auf, welche bei gegenüber der Herzoberfläche zurückbleibender Bewegung einerseits zu einer unzureichenden Kontaktierung des Herzmuskels und andererseits zu einer Behinderung der Herzbewegung führt, da die betreffenden Elektroden meist am Herzen festgenäht sind.

Weiterhin ist aus der EP 0 211 166 A2 eine Elektrode bekannt, welche aus mehreren stern- oder strahlenförmigen Elektrodenarmen besteht, die an der Herzoberfläche befestigt werden. Hierbei besteht nun wiederum der Nachteil, daß die Befestigung der einzelnen Elektrodenarme relativ zeitraubend ist. Auch entspricht die örtliche Befestigung vielfach nicht der bei der Defibrillation tatsächlich notwendigen zu erzielenden Stromverteilung.

Eine Elektrode mit verbesserten Eigenschaften ist aus EP-A-0 115 778 bekannt, ein gattungsgemäßes Defibrillationssystem aus EP-A-0 453 117.

Nachteilig bei den bekannten Elektroden ist, daß trotzdem noch relativ große Energien zur Defibrillation erforderlich sind, so daß entweder sehr große Energiespeicher innerhalb des implantierbaren Gehäuses vorgesehen sein müssen - wodurch die Implantation wegen seines großen Volumens letztlich nicht wie die eines heutigen Herzschrittmachers gehandhabt werden kann - oder aber bei häufiger Inanspruchnahme der Defibrillationsfunktion mit vorzeitiger Erschöpfung der Energiespeicher gerechnet werden muß. Letzteres ist insbesondere bei solchen Geräten nachteilig, bei denen das Defibrillationsgerät mit einem üblichen Herzschrittmacher zusammengefaßt ist, da dann bei Erschöpfung der Batterie keine der löebenserhaltenden Funktionen mehr zur Verfügung steht.

Der Erfindung liegt die Aufgabe zugrunde, bei einem implantierbaren Defibrillationssystem der eingangs genannten Gattung die mögliche Betriebszeit bis zur Erschöpfung der eingebauten Energiequelle durch Verringerung des Energiebedarfs weiter heraufzusetzen.

Diese Aufgabe wird mit den kennzeichnenden Merkmalen des Anspruchs 1 gelöst.

Die Erfindung schließt die Erkenntnis ein, daß die Elektrodenoberfläche als Schnittstelle zu dem zu defibrillierenden Gewebe und damit dem verwendeten Werkstoff eine besondere Bedeutung zukommt. Während bisher die in das Gewebe überzuleitende Defibrillationsenergie hauptsächlich durch die Impedanz der Elektrodenoberfläche bestimmt wurde und damit auch - bei vergleichbaren Impulsamplituden - der im Gewebe wirksame Energieanteil relativ gering war, ist es mit der neuartigen Ausführung der Elektrodenoberflächen nach der Erfindung so, daß der Übergangswiderstand an der Elektrodenoberfläche klein ist gegenüber dem Gewebewiderstand. Damit steht ein höherer Spannungsanteil für die Derfibrillation zur Verfügung, so daß - entsprechend der sich im Gewebe einstellenden Strom- bzw. Feldverteilunggrößere Gewebebereiche für die Defibrillation erreichbar sind. Damit wird erreicht, daß auch mit Elektroden kleinerer Oberfläche Defibrillationswirkungen in Gewebezonen erreicht werden können, die bisher wesentlich größere Elektrodenoberflächen erforderten.

Durch die Möglichkeit, die Polarität, d.h. die Richtung des Difibrillationsstroms, bei den angeschlossenen Defibrillationselektroden zu ändern, kann die Erfolgsrate der Defibrillationen patientenindividuell noch heraufgesetzt werden.

Die Erfindung schließt die Erkenntnis ein, daß die Werkstoffe der bekannten Elektroden und insbesondere Titan, Vanadium, Zirkon und Niob zu teilweise extremer Oxidation neigen und daß diese hohe Oxidationsneigung bei Kontakt zu wässrigen Elektrolyten dazu führt, daß sich an der Elektrodenoberfläche eine dünne, isolierende bzw. halbleitende Oxidschicht bildet, die eine der Helmholtzkapazität C_{H} in Serie geschaltete Kapazität Cₒₓ darstellt und so zur langsamen Verringerung der Gesamtkapazität und damit zur entsprechenden Erhöhung der jeweils erforderlichen Defibrillationsenergie führt. Bei anodischer Polung werden OH⁻-Ionen in den Festkörper gezogen und führen dort zur Vergrößerung der Oxidschichtdicke. Dies hat eine weitere Verringerung der Phasengrenzkapazität und damit eine weitere Erhöhung der Elektrodenimpedanz zur Folge.

Damit ist mit den herkömmlichen beschichteten porösen Elektroden wegen ihrer großen relativen Oberfläche zunächst grundsätzlich eine Defibrillation mit gutem Erfolg bei niedriger Energie möglich. Es wurde nun erkannt, daß durch die Oxidationsneigung die Helmholtzkapazität verkleinert wird, was zu einer Erhöhung der Elektrodenimpedanz führt. Die damit hervorgerufene Beeinflussung der Elektrodeneigenschaften im Laufe der Implantationszeit ist deshalb so schwerwiegend, weil die Verschlechterung der Elektrodeneigenschaften Auswirkungen hat, welche ihrerseits dazu beitragen, daß die Defibrillationseigenschaften zusätzlich ungünstig beeinflußt werden.

Die langzeitstabile, bioverträgliche Oberflächenbeschichtung der erfindungsgemäßen Defibrillationselektrode besteht aus einem Material, dessen Oxidationsneigung sehr gering ist, wobei sie vorzugsweise unter Verwendung eines inerten Materials, also eines Nitrides, Carbides, Carbonitrides oder aber eines reinen Elementes bzw. bestimmter Legierungen aus der Gruppe der Platinwerkstoffe: Platin, Iridium und Tellur vakuumtechnisch auf die Elektrode aufgetragen wird. Wegen der fraktalen räumlichen Geometrie einer derart aufgetragenen Oberflächenschicht ist deren aktive Oberfläche sehr groß, so daß die zur Defibrillation erforderliche Energiemenge gering gehalten werden kann.

Die Oberflache der Elektrode kann mindestens über einen Abschnitt ihrer Länge Drähten oder Faserbündeln in einer regulären Anordnung aufweisen derart, daß die Elektrode in diesem Bereich eine wesentlich größere Oberfläche aufweist, als es ihrer geometrischen Grundform entspricht.

Die Vorteile des erfindungsgemäßen Defibrillationssystems liegen insbesondere in der großen erzielbaren Kapazität der Elektrode, welche widerum eine wesentliche Energieersparnis bei der Defibrillation mit sich bringt. Diese ist um so mehr von Bedeutung, als bei implantierbaren Systemen der Energieverbrauch wegen der ebenfalls in dem zu implantierenden Gehäuse vorgesehenen Energiespeicher bzw. Batteriezellen besonders niedrig gehalten werden sollte, um den Patienten nicht mit einem zu großen Gehäusevolumen zu belasten. Vorteilhaft ist eine wirksame Oberfläche der Elektrode von ca. 100 cm². Die bei einem Defibrillationsschock abgegebene Energie beträgt bei dem erfindungsgemäßen System nur rund 400 Ws. Besonders vorteilhaft wirkt sich auch der Umstand aus, daß die Defibrillationsimpulse mit beiden Polaritäten abgegeben werden können, da Oxidations- bzw. Redoxreaktionen an der Elektrodenoberfläche nicht vorkommen. Auf diese Weise lassen sich auch die Rückladungsimpulse eines Ausgangs-Koppelkondensators zur Defibrillation nutzen, was wiederum zur weiteren Energieersparnis beiträgt.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein erstes Ausführungsbeispiel der erfindungsgemäßen Defibrillations- und Schrittmacherelektrode zur Verlegung im rechten Ventrikel,
Figur 2 ein weiteres Ausführungsbeispiel der erfindungsgemäßen Elektrode zur subcutanen bzw. epicardialen Anwendung sowie
Figur 3 ein implantierbares Steuerteil für ein erfindungsgemäßes Defibrillationssystem in schematischer Darstellung.

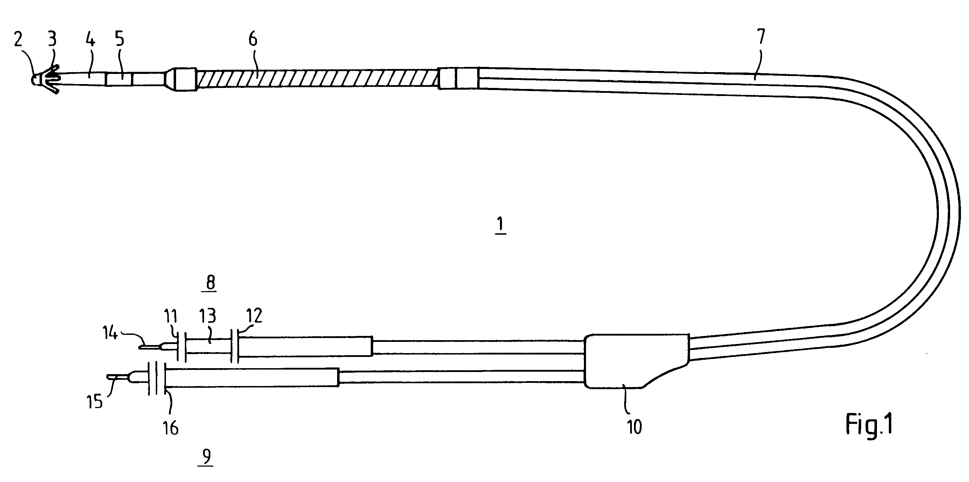

Die in Figur 1 dargestellte kombinierte Defibrillator-Schrittmacherelektrode 1 besteht aus einem üblichen Stimulationskopf, der an seiner distalen Spitze eine Stimulations- und Sensingfläche 2 aufweist und Befestigungselemente 3, welche gegenüber der Stimulations- und Sensingfläche geringfügig zurückversetzt sind. Anschließend an einen isolierenden Bereich 4 folgt eine Bezugselektrode 5 für das bipolar ausgebildete Stimulationssystem. Ungefähr 25 mm gegenüber der Spitze zurückversetzt schließt sich dann eine Schockwendel 6 an, welche sich über einen Bereich von 45 mm der Länge des distalen Elek-trodenendes erstreckt. Der Außendurchmesser der Schockwendel, die aus einem Draht mit im wesentlichen 0,2 mm Durch-messer aus PtIr 80/20 besteht, beträgt ca. 3 mm. Die Oberfläche der Schockwendel ist mit einer Iridiumschicht großer Oberfläche versehen, welche eine fraktale Oberflächengeometrie aufweist. Anschließend an die Schockwendel folgt die isolierte Elektrodenzuleitung 7, in der mehrere koaxiale Wendeln verlaufen, die jeweils die Kontaktbereiche 2, 4 und 6 mit entsprechenden Kontaktbereichen von Steckerelementen 8 und 9 galvanisch verbinden. Die Zuleitung selbst ist dabei noch von einem Isoliermantel umgeben. Am proximalen Ende ist ein Verzweigungsbereich 10 vorgesehen, welcher in einen Schrittmacherstecker (Steckerelement 8) mündet, der zwischen zwei umlaufenden radial gerichteten Dichtungen 11 und 12 einen Kontaktbereich 13 aufweist, der mit der Bezugselektrode 5 verbunden ist.

Das freie Steckerende 14 ist hingegen mit dem stimulierenden und sensenden Bereich 2 des distalen Endes verbunden. Die Schockwendel 6 ist hingegen mit einem freien Steckerende 15 des Steckerelementes 9 verbunden, wobei ein elastischer, in radialer Richtung wirksamer umlaufender Dichtungsbereich 16 vorgesehen ist, um entsprechend den Dichtungsbereichen 11 und 12 eine Abdichtung der Kontaktbereiche gegen Körperflüssigkeit herbeizuführen, wenn die Steckerelemente in den Schrittmacher eingesetzt sind.

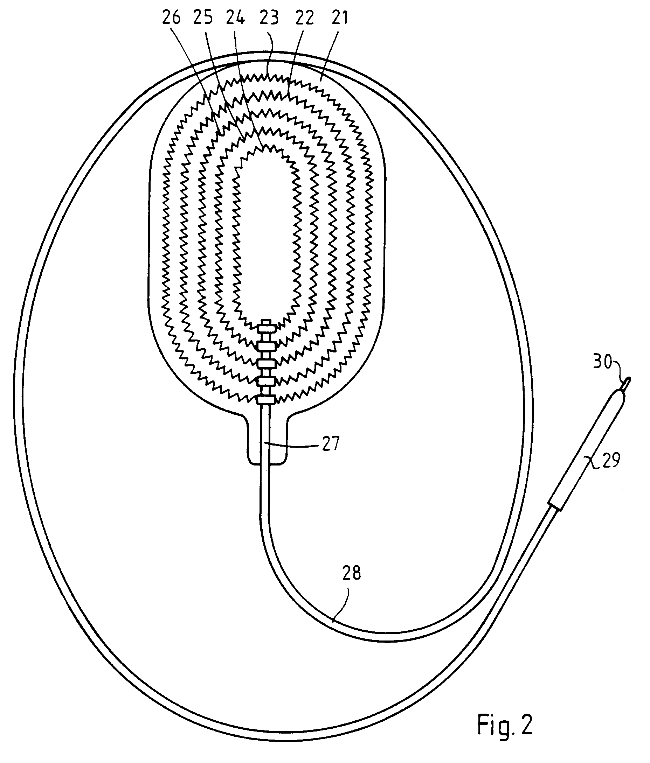

In Figur 2 ist eine weitere Elektrode dargestellt, welche an ihrem distalen Ende einen flächenhaft ausgestalteten Kontaktierungsbereich aufweist und zum Befestigen am Epicard bestimmt ist. Bei alternativer Verwendung kann diese Elektrode auch subcutan appliziert werden. Hierbei ist eine Wendel aus einer Platin-Iridium-Legierung (PtIr 80/20) in ein Substrat aus einem körperverträglichen, elastischen Isolierwerkstoff in der Weise eingebettet, daß der größte Teil der ovalförmig konzentrisch verlaufenden Wendeln 22 bis 26 zur elektrischen Kontaktierung von extern zugänglich ist. Die Befestigung der Wendeln im Substratwerkstoff erfolgt dabei in der Weise, daß jeweils ein gleichsinnig gelegener Teil ihres Umfangsbereiches von dem Werkstoff des Substrats umhüllt ist, was beispielsweise durch Umgießen, Umspritzen oder dergleichen erfolgen kann. Die Oberfläche des Elektrodenleiters ist ebenfalls mit Iridium beschichtet und es wird insoweit auf die diesbezüglichen anderen diese Beschichtung beschreibenden Teile der Beschreibung verwiesen.

Während die beiden äußerern ovalartigen Ringe 22 und 23 als Doppelwendeln ausgestaltet sind, sind die drei inneren Wendeln 24 bis 26 des dargestellten Ausführungsbeispiels als Einfachwendeln konzipiert. Sämtliche konzentrischen ovalen Wendeln 22 bis 26 kontaktieren eine den Verlauf der Wendein in der Mitte der halbkreisförmigen Bogen der ovale an einer Seite kreuzenden Verbindungsleitung 27, welche ihrerseits mit einer vollständig mit Isolierstoff ummantelten Zuleitung 28 verbunden ist, in der die Zuleitungswendel verläuft und zu einem Steckerlement 29 hinführt, dessen freier Steckerstift 30 zum Anschluß dieser Elektrode am Defibrillationsteil des Schrittmachers dient.

Eine weitere bei dem hier zu beschreibenden Stimulationssystem verwendete Elektrode zur Applikation in der Vena cava superior ist nicht näher dargestellt, in ihrer Konzeption entspricht sie aber der Ausführung gemäß Figur 1, wobei allerdings der stimulierende Teil entfallen kann, so daß insoweit eine Schockwendel vorgesehen ist, die das distale Ende einer derartigen Elektrode bildet und über eine Zuleitung unmittelbar mit einem Steckerelement 9 verbunden ist, so daß sowohl auf die stimulierende und sensende Fläche als auch auf das zur Kontaktierung dieser Fläche am Schrittmacher vorgesehene Steckerelement 8 verzichtet werden kann.

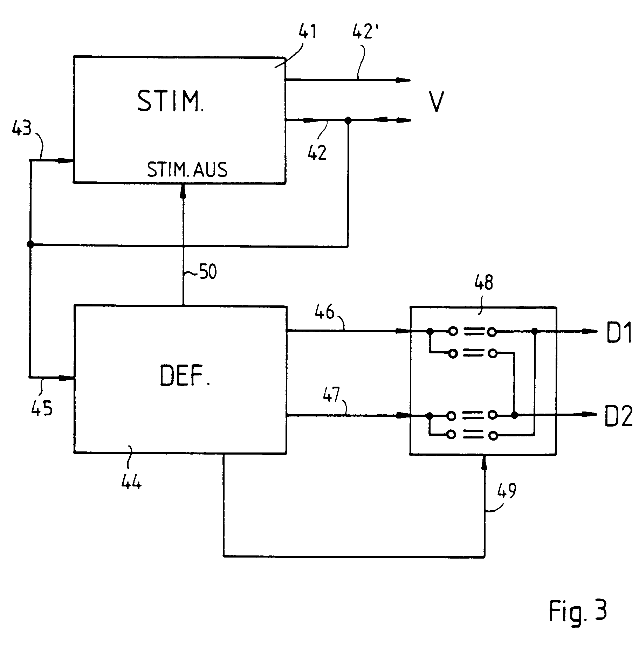

In Figur 3 ist in Blockschaltung ein implantierbarer Schrittmacher/Defibrillator dargestellt, welcher in Verbindung mit den zuvor dargestellten Elektroden verwendbar ist. Ein herkömmlicher Schrittmacher 41 weist einen Anschluß 42 (sowie bei bipolarer Stimulation den zusätzlich Eingang 42' für das Referenzpotential) und einen Senseeingang 43 auf, mit dem Stimulationsimpulse an den Ventrikel abgebbar bzw. elektrische im Herzen selbst hervorgerufenen elektrische Signalereignisse aufnehmbar sind. Zusätzlich zu dem Schrittmachersystem 41 ist ein Defibrillationssystem 44 vorgesehen, dem über einen entsprechenden Eingang 45 ebenfalls die im Ventrikel aufgenommenen elektrischen Signale zugeführt werden. Ausgangsseitig weist das Defibrillationssystem 44 zwei Signalausgänge 46 und 47 auf, an denen hochenergetische Impulse für die Defibrillationselektroden abnehmbar sind. Den beiden Ausgängen 46 und 47 ist ein Umschalter 48 nachgeschaltet, der interne Schaltmittel aufweist, mit dem auf ein entsprechendes Eingangssignal 49 hin die Anschlüsse der Defibrillationselektroden D1 und D2 vertauscht werden können, so daß eine Erhöhung der Erfolgsaussicht der Defibrillation durch Wahl der für den Patienten günstigsten individuellen Polarität des Elektrodenanschlusses möglich ist.

Die Umpolschaltung 48 wird durch einen entsprechenden Ausgang des Defibrillationssystems über eine Signalleitung 49 gesteuert, wobei eine Aktivierung dieser Signalleitung die Vertauschung der beiden Anschlüsse D1 und D2 bewirkt.

Eine Defibrillation wird ausgelöst, wenn über den Eingang 45 Kammerflimmern detektiert wird. In diesem Fall wird zusätzlich ein Ausgangssignal über eine Leitung 50 ausgegeben, welche zusätzlich die Ausgabe von Stimulationsimpulsen durch das Schrittmachersystem 41 auf die Leitung 42 unterbindet.

## Patentansprüche

1. Implantierbares Defibrillationssystem mit einer intrakardialen oder subcutanen Defibrillationselektrode, die mindestens abschnittsweise mit einer porösen Oberflächenbeschichtung aus einem inerten Element, einer inerten chemischen Verbindung und/oder einer inerten Legierung, deren aktive Oberflache wesentlich größer ist als die sich aus der geometrischen Grundform der Elektrode ergebende Oberfläche, versehen ist, **dadurch gekennzeichnet,** daß die aktive Oberfläche durch eine fraktalartige räumliche Geometrie um einen Faktor von mindestens tausend größer ist als die sich aus der geometrischen Grundform der Elektrode ergebende Oberfläche.

2. Defibrillationssystem nach Anspruch 1, **dadurch gekennzeichnet,** daß das inerte Material Kohlenstoff, ein Nitrid, Carbid oder Carbonnitrid oder ein reines Element, eine Legierung oder eine Verbindung von Elementen aus der Gruppe Gold, Silber, Platin, Iridium und Tellur ist.

3. Defibrillationssystem nach Anspruch 1, **dadurch gekennzeichnet,** daß die Beschichtung aus Iridiumnitrid besteht.

4. Defibrillationssystem nach einem der vorangehenden Ansprüche, **dadurch gekennzeichnet,** daß die Oberflächenbeschichtung mittels Dünnschichttechnologie auf die Elektrode aufgebracht, insbesondere aufgedampft, ist.

5. Defibrillationssystem nach Anspruch 4, **dadurch gekennzeichnet,** daß die Oberflächenbeschichtung mittels reaktiver Kathodenzerstäubung (CVD, PVD oder MOCVD) oder Ionenplattierung auf die Elektrode aufgebracht ist.

6. Defibrillationssystem nach einem der vorangehenden Ansprüche, **dadurch gekennzeichnet,** daß die Elektrode einen Grundkörper aus Titan aufweist.

7. Defibrillationssystem nach einem der Ansprüche 1 bis 6, **dadurch gekennzeichnet,** daß die Oberfläche der Elektrode mindestens über einen Abschnitt ihrer Länge Drähten oder Faserbündeln in einer regulären Anordnung aufweist derart, daß die Elektrode in diesem Bereich eine wesentlich größere Oberfläche aufweist, als es ihrer geometrischen Grundform entspricht.

8. Defibrillationssystem nach Anspruch 7, **dadurch gekennzeichnet,** daß der Drähte oder Faserbündel aufweisende Abschnitt der Elektrodenoberfläche mehrere Drähte oder Faserbündel unterschiedlicher Dicke aufweist, wobei jeweils Drähte oder Faserbündel unterschiedlicher Dicke einander benachbart sind.

9. Defibrillationssystem nach Anspruch 7 oder 8, **dadurch gekennzeichnet,** daß die Drähte oder Faserbündel, vorzugsweise um einen flexiblen Kern, koaxial gewendelt sind.

10. Defibrillationssystem nach Anspruch 9, **dadurch gekennzeichnet,** daß der Drähte oder Faserbündel aufweisende Abschnitt der Elektrodenoberfläche eine Doppelwendelstruktur aufweist.

11. Defibrillationssystem nach einem der Ansprüche 6 bis 10, **dadurch gekennzeichnet,** daß der Drähte oder Faserbündel aufweisende Abschnitt der Elektrodenoberfläche eine spiralige, mäanderförmige oder aus Kreisen unterswchiedlichen Durchmessers gebildete Anordnung der Drähte oder Faserbündel mit insbesondere kreisförmiger, ovaler oder mattenförmiger Außenkontur aufweist.

12. Defibrillationssystem nach einem der vorangehenden Ansprüche, **dadurch gekennzeichnet,** daß die Elekrode als Myocard- oder Epicardelektrode oder Einführung durch die vena cava superior ausgestaltet ist.

13. Defibrillationssystem nach einem der vorangehenden Ansprüche, **dadurch gekennzeichnet,** daß die Elektrode gleichzeitig mindestens Teil einer Schrittmacherelektrode ist.

## Claims

1. An implantable defibrillation system comprising an intracardial or subcutaneous defibrillation electrode provided, at least in sections, with a porous surface coating of an inert element, an inert chemical compound and/or an inert alloy, the active surface of which coating is considerably larger than the surface determined by the basic geometrical shape of the electrode, characterised in that, by virtue of a fractal-type spatial geometry, the active surface is larger than the surface determined by the basic geometrical shape of the electrode by a factor of at least a thousand.

2. A defibrillation system according to claim 1, characterised in that the inert material is carbon, a nitride, carbide or carbonic nitride or a pure element, an alloy or a compound of elements from the group comprising gold, silver, platinum, iridium and tellurium.

3. A defibrillation system according to claim 1, characterised in that the coating is of iridium nitride.

4. A defibrillation system according to one of the preceding claims, characterised in that the surface coating is applied to the electrode by means of thin-layer technology, particularly by vapour deposition.

5. A defibrillation system according to claim 4, characterised in that the surface coating is applied to the electrode by means of reactive cathode sputtering (CVD, PVD or MOCVD) or ionic plating.

6. A defibrillation system according to one of the preceding claims, characterised in that the basic body of the electrode is made of titanium.

7. A defibrillation system according to one of claims 1 to 6, characterised in that the surface of the electrode has wires or bunches of fibre in a regular arrangement at least over a section of its length, in such a way that the electrode has a considerably larger surface in that area than would correspond to its basic geometrical shape.

8. A defibrillation system according to claim 7, characterised in that the section of the electrode surface with wires or bunches of fibre has a plurality of wires or bunches of fibre of different thickness, in each case with wires or bunches of fibre of different thickness adjacent to one another.

9. A defibrillation system according to claim 7 or 8, characterised in that the wires or bunches of fibre are coiled coaxially, preferably around a flexible core.

10. A defibrillation system according to claim 9, characterised in that the section of the electrode surface with wires or bunches of fibre has a double coil structure.

11. A defibrillation system according to one of claims 6 to 10, characterised in that the section of the electrode surface with wires or bunches of fibre has the wires or bunches of fibre in an arrangement which is spiral, meandering or formed by circles of different diameter, particularly with a circular, oval or mat-like external profile.

12. A defibrillation system according to one of the preceding claims, characterised in that the electrode is constructed as a myocardial or epicardial electrode or an entrance through the vena cava superior.

13. A defibrillation system according to one of the preceding claims, characterised in that the electrode is at the same time at least part of a pacemaker electrode.

## Revendications

1. Système de défibrillation implantable équipé d'une électrode de défibrillation intracardiaque ou sous-cutanée, qui est muni au moins sous forme de tronçon, d'un revêtement superficiel poreux en un élément inerte, en un composé chimiquement inerte et/ou en un alliage inerte, dont la surface active est sensiblement supérieure à la surface s'obtenant de la forme géométrique fondamentale de l'électrode, caractérisé en ce que la surface active à travers une géométrie spatiale fractionnaire est plus grande d'un facteur d'au moins 1000 que la surface résultant de la forme géométrique fondamentale de l'électrode.

2. Système de défibrillation selon la revendication 1, caractérisé en ce que le matériau inerte est du carbone, un nitrure, un carbure ou un nitrure de carbone ou un élément pur, un alliage ou un composé d'éléments appartenant au groupe or, argent, platine, iridium et tellure.

3. Système de défibrillation selon la revendication 1, caractérisé en ce que le revêtement est en nitrure d'iridium.

4. Système de défibrillation selon l'une quelconque des revendications précédentes, en ce que le revêtement superficiel est déposé sur l'électrode par technologie des couches minces, en particulier évaporé.

5. Système de défibrillation selon la revendication 4, caractérisé en ce que le revêtement superficiel est déposé par pulvérisation cathodique réactive (CVD, PVD ou MOCVD) ou revêtement ionique sur l'électrode.

6. Système de défibrillation selon l'une quelconque des revendications précédentes, caractérisé en ce que l'électrode présente un corps fondamental en titane.

7. Système de défibrillation selon l'une quelconque des revendications 1 à 6, caractérisé en ce que la surface de l'électrode présente au moins sur un tronçon de sa longueur des fils ou des faisceaux de fibres disposés régulièrement, de telle sorte que l'électrode présente dans cette région une surface sensiblement plus grande que celle qui correspond à sa forme géométrique fondamentale.

8. Système de défibrillation selon la revendication 7, caractérisé en ce que le tronçon de la surface de l'électrode présentant des fils ou un faisceau de fibres comporte plusieurs fils ou faisceaux de fibres d'épaisseur différente, des fils ou des faisceaux de fibres d'épaisseur différente étant respectivement adjacents les uns aux autres.

9. Système de défibrillation selon la revendication 7 ou 8, caractérisé en ce que les fils ou faisceaux de fibres sont bobinés coaxialement de préférence autour d'une âme flexible.

10. Système de défibrillation selon la revendication 9, caractérisé en ce que le tronçon de la surface d'électrode présentant des fils ou des faisceaux de fibres présente une structure en hélice double.

11. Système de défibrillation selon l'une quelconque des revendications 6 à 10, caractérisé en ce que le tronçon de la surface de l'électrode présentant des fils ou des faisceaux de fibres présente une disposition des fils ou des faisceaux de fibres formée en spirale, en méandres ou en cercle de diamètre différent, avec un contour extérieur, en particulier circulaire, ovale ou en forme de nattes.

12. Système de défibrillation selon l'une quelconque des revendications précédentes, caractérisé en ce que l'électrode est réalisée en tant qu'électrode de myocarde ou d'épicarde ou d'introduction à travers la veine cave supérieure.

13. Système de défibrillation selon l'une quelconque des revendications précédentes, caractérisé en ce que l'électrode est simultanément au moins une partie d'une électrode d'un stimulateur cardiaque.
